# EUROPEAN PATENT APPLICATION

(11) **EP 4 721 683 A1**
(43) Date of publication of application: **08.04.2026**
(21) Application number: 23938508.1
(22) Date of filing: 24.05.2023
(51) Int. Cl.: A61B 17/70

(54) **TREATMENT INSTRUMENT**

(71) Applicant: Spine Chronicle Japan Co., Ltd., Kanazawa-city, Ishikawa 920-0848 (JP)
(72) Inventor: YONEZAWA Noritaka, Kanazawa-city Ishikawa 920-0848 (JP)
(74) Representative: Plougmann Vingtoft a/s
(86) International application number: PCT/JP2023/019387
(87) International publication number: WO 2024/241545

(57) **Abstract**

A treatment device comprising a trial cage, a bone drill needle, and a bone cement filling tube, wherein the trial cage has a head of a predetermined thickness, width, and length, and a support rod that supports the head at the longitudinal end of the head. the head has a flat surface that defines the predetermined thickness and at least one guide hole, the guide hole penetrates the head in the thickness direction from the distal side of the head toward one of a flat surfaces and allows passage of respective tip sections of the bone drill needle and the bone cement filling tube. A treatment device used in procedures prior to the insertion of an artificial intervertebral disc into an intervertebral disc space that is capable of forming bone tunnels in vertebrae above and below the artificial intervertebral disc, filling bone cement through those tunnels while the same device is inserted, and reinforcing the vertebrae above and below a cage can be provided.

## Description

### Technical Field

The present invention relates to a treatment device for a spine and a cervical spine which inserts an intervertebral spinal implant (i.e., an artificial intervertebral disc) into an intervertebral disc space, and is particularly suitable for use in surgery to insert it between vertebrae (also called "vertebral body") before the artificial intervertebral disc is inserted to secure a gap (i.e., intervertebral disc space) for insertion.

### Background Art

One treatment for the spine is the surgical implantation of a specialized artificial intervertebral disc material (sometimes called a "cage," but referred to herein as an "artificial intervertebral disc") between adjacent vertebrae (i.e., intervertebral disc space). While adjacent vertebrae articulate with the intervertebral disc between them through thin cartilage called endplate in a healthy state, when the disease that damages the disc becomes severe, the application of surgery to implant an artificial intervertebral disc is considered. In this procedure, an artificial intervertebral disc is inserted and implanted into the gap (i.e., intervertebral disc space) formed by removing the damaged disc. The inserted artificial intervertebral disc fuses with the vertebrae to fix the intervertebral space. A trial cage (sometimes referred to simply as a "trial" or "cage trial," but referred to herein as a "trial cage") is utilized to determine the appropriate size of the artificial intervertebral disc to be inserted. Trial cages are prepared in various sizes and inserted into the intervertebral space to identify the appropriate size.

Currently, the demand for spinal fusion surgery for elderly people is increasing in Japan. Spinal fusion is a treatment method in which a specialized artificial intervertebral disc is inserted into a narrowed intervertebral space (i.e., intervertebral disc space) and fill the surrounding area with autologous and artificial bone tissue to fuse two vertebrae that sandwich the artificial intervertebral disc. When the gap between the vertebrae narrowed by the artificial intervertebral disc is restored to its normal height, and bony fusion is achieved with the height maintained, symptoms such as neuralgia and back pain can be improved.

PTL 1 discloses a trial consisting of a shaft and an end attached to the end of the shaft and inserted into the gap between the vertebrae. The shaft is hollow and has a passage and an outlet from its end to the outside of the end. Various bone growth promoting substances such as bone-forming proteins, bone graft tissue, bone meal, and bone cement can be introduced into the affected area through the shaft and an outlet at the end, and also access to the affected area for instruments such as extruders, impact devices, and delivery tubes is provided (paragraph 0009, etc.).

PTL 2 discloses a endplate drill that can form a bone tunnel that is generally perpendicular to the endplate. It is configured to allow the needle member to protrude almost perpendicularly into the vertebral body from the actuator case, which is inserted into the gap between the vertebrae, and is said to safely and easily promote bone fusion between the vertebrae.

### CITATION LIST

### Patent Document

PTL 1: Japanese Patent Application Laid-Open No. 2018-69067
PTL 2: Japanese Patent Application Laid-Open No. 2022-52575

### SUMMARY OF THE INVENTION

### Problems to be Solved by the Invention

The inventor realized that elderly people often have osteoporosis in their backgrounds, and that conventional artificial intervertebral disc implantation surgery runs the risk of the phenomenon of the artificial intervertebral disc digging into the endplates of the upper and lower vertebrae that sandwich the artificial intervertebral disc (i.e., fracture of the endplate bone and settling of the artificial intervertebral disc). This makes it difficult to maintain the narrowed intervertebral space at a normal height until bony fusion, and as a result, not only is there no improvement in symptoms, but it is not uncommon for major revision surgery to be required due to loosening of the implants. The present inventor's challenge was to create a technique that would allow the vertebrae in contact above and below the artificial intervertebral disc to be reinforced with bone cement, so that the artificial intervertebral disc could maintain the size of the intervertebral disc space without settling into the vertebrae. The term "above and below" is an expression that assumes the patient is standing and the spine is generally vertical from the floor (the same is true throughout this specification, unless otherwise noted). The vertebrae are stacked vertically across the intervertebral discs to form the spine. The expression does not intend to mean above and below based on the posture of the patient during surgery.

The trial cage shown in PTL 1 maintains the reinforcement and size of the intervertebral disc space in that a bone growth promoting substance can be introduced with the trial cage inserted. The trial cage is also said to be able to pass through extruders, impact devices, delivery tubes, and other instruments (paragraph 0009). However, such devices "can be desirable when bone quality has deteriorated and/or there is a void to be filled and the surgeon seeks to densify the vertebral body bone and/or fill the void with graft tissue material to bring about a firmer surface for the intervertebral spinal implant to seat" (0016 paragraph), which only reinforces the intervertebral disc space.

The endplate drill shown in Document 2 is safe and easy to project generally perpendicular to the vertebral body, while the shaft is provided, within the actuator case, with a mechanical structure for changing the direction of force transmission in order to project perpendicularly as well, making it possible to drill holes in the upper and lower vertebrae that make up the intervertebral disc space. However, it does not take into account the introduction of bone cement or bone growth stimulants through the formed bone tunnel with the same device inserted.

An object of the present invention is to provide a treatment device used in procedures prior to the insertion of an artificial intervertebral disc into an intervertebral disc space that is capable of forming bone tunnels in vertebrae above and below the artificial intervertebral disc, filling bone cement through those tunnels while the same device is inserted, and reinforcing the vertebrae above and below the artificial intervertebral disc.

Means for solving these problems will be described below, but other issues and novel features will become apparent from the description herein and the accompanying drawings.

### Means for Solving Problems

According to one embodiment of the present invention, a means for solving the problems is as follows.

That is, a treatment device comprising a trial cage, a bone drill needle, and a bone cement filling tube, wherein the trial cage has a head of a predetermined thickness, width, and length, and a support rod that supports the head at the longitudinal end of the head. The head has a flat surface that defines the predetermined thickness and at least one guide hole, the guide hole penetrates the head in the thickness direction from the distal side of the head toward one of a flat surfaces and allows passage of respective tip sections of the bone drill needle and the bone cement filling tube.

### Effect of the Invention

The effect to be produced by the above-described embodiment will be briefly described below.

That is, a treatment device used in procedures prior to the insertion of an artificial intervertebral disc into an intervertebral disc space that is capable of forming bone tunnels in vertebrae above and below the artificial intervertebral disc, filling bone cement through those tunnels while the same device is inserted, and reinforcing the vertebrae above and below the artificial intervertebral disc from inside can be provided.

### BRIEF DESCRIPTION OF THE DRAWINGS

FIG. 1 is an explanatory drawing illustrating an example configuration of a treatment device, a first embodiment according to the present invention.
FIG. 2 is an enlarged view illustrating a tip section of a trial cage according to the present invention.
FIG. 3 is an explanatory drawing illustrating an example configuration of the tip section of the trial cage according to the present invention.
FIG. 4 is an explanatory drawing illustrating in perspective an action of the tip section of the trial cage according to the present invention.
FIG. 5 is an explanatory drawing illustrating an example configuration of a treatment device, a second embodiment according to the present invention.

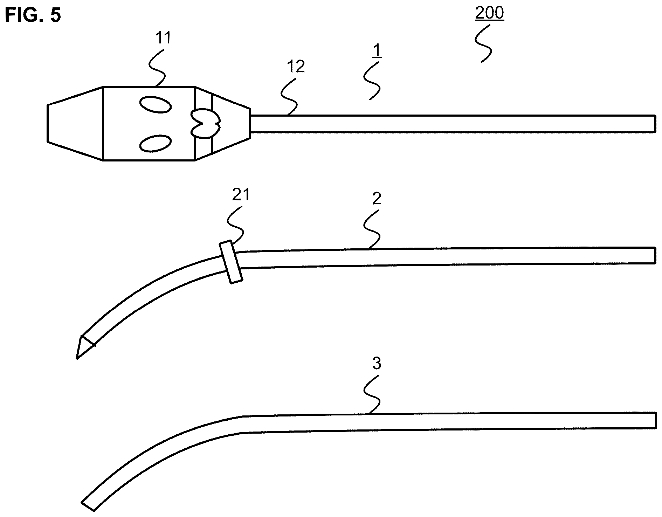

FIG. 5 is an explanatory drawing illustrating in perspective an action of the tip section of the trial cage of the second embodiment.

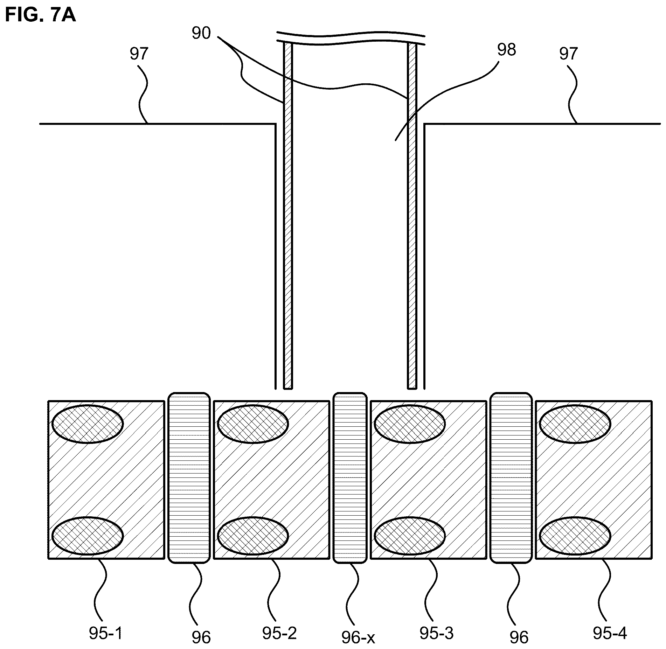

FIG. 7a is an explanatory drawing illustrating a first step (incision) of a surgical example using the treatment device according to the present invention.

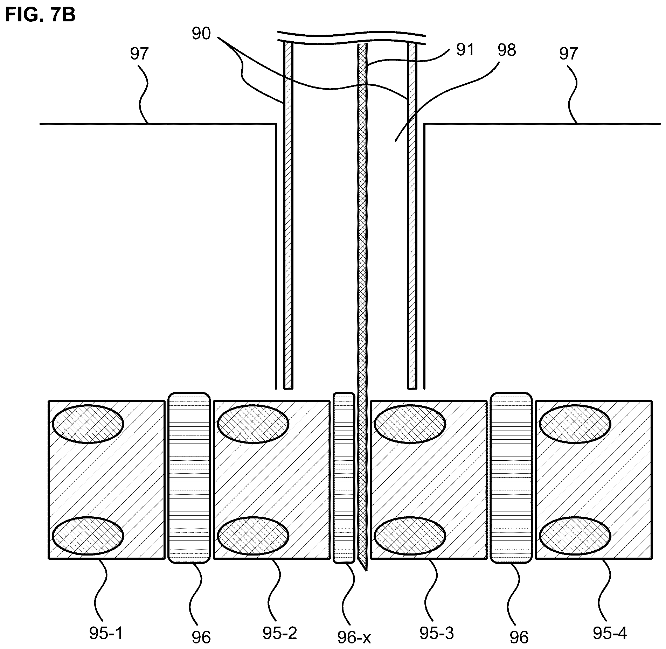

FIG. 7B is an explanatory drawing illustrating the second step (intervertebral disc removal) of a surgical example using the treatment device according to the present invention.

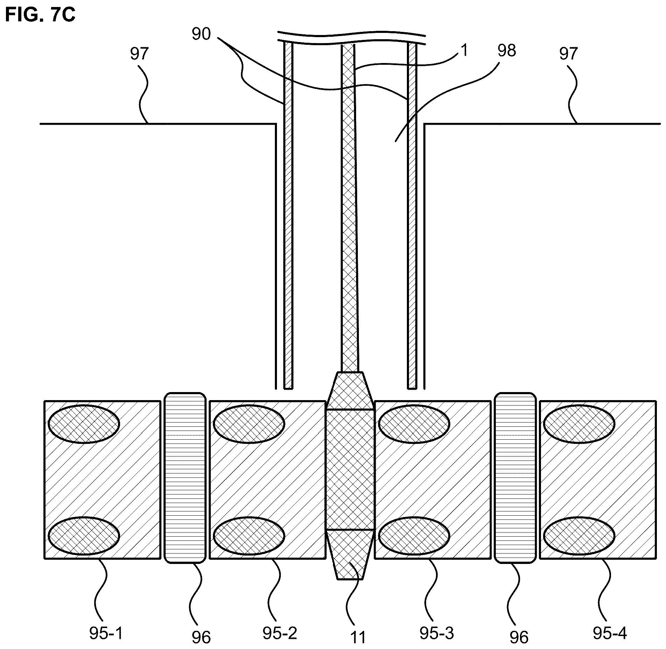

FIG. 7C is an explanatory drawing illustrating the third step (trial cage insertion) of a surgical example using the treatment device according to the present invention.

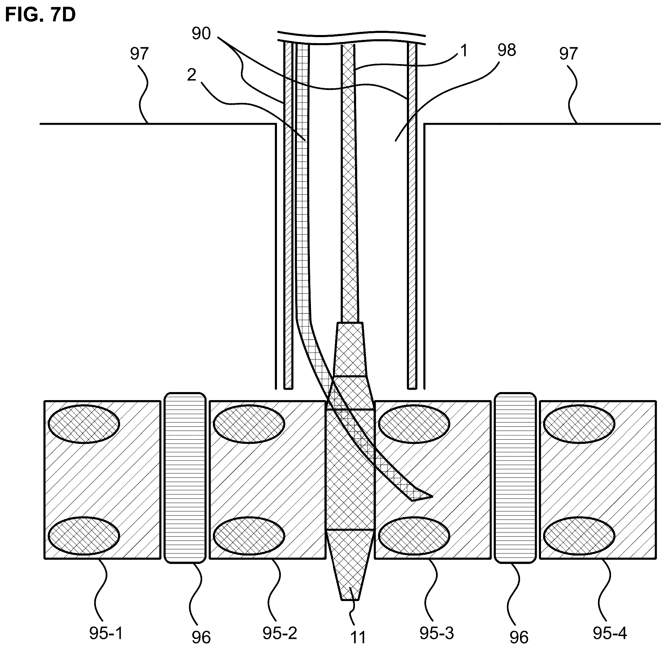

FIG. 7D is an explanatory drawing illustrating the fourth step (drilling) of a surgical example using the treatment device according to the present invention.

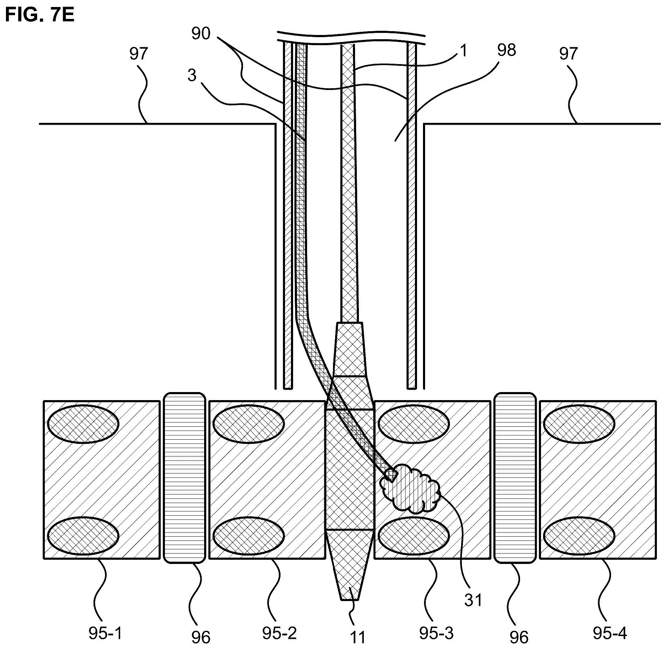

FIG. 7E is an explanatory drawing illustrating the fifth step (filling of bone cement) of a surgical example using the treatment device according to the present invention.

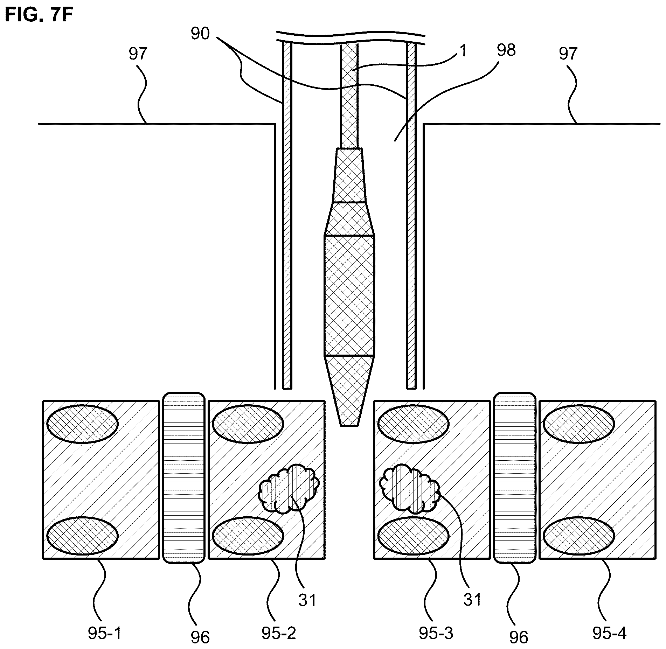

FIG. 7F is an explanatory drawing illustrating the sixth step (removal of trial cage) of a surgical example using the treatment device according to the present invention.

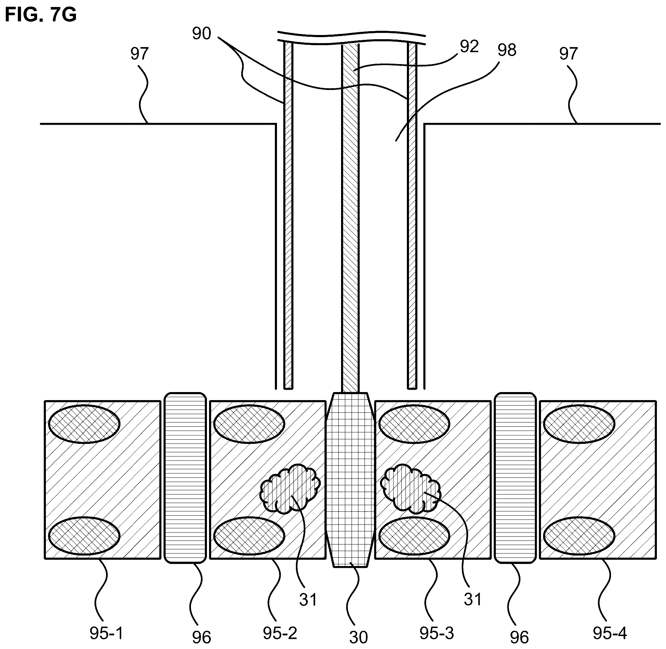

FIG. 7G is an explanatory drawing illustrating the seventh step (insertion of artificial intervertebral disc) of a surgical example using the treatment device according to the present invention.

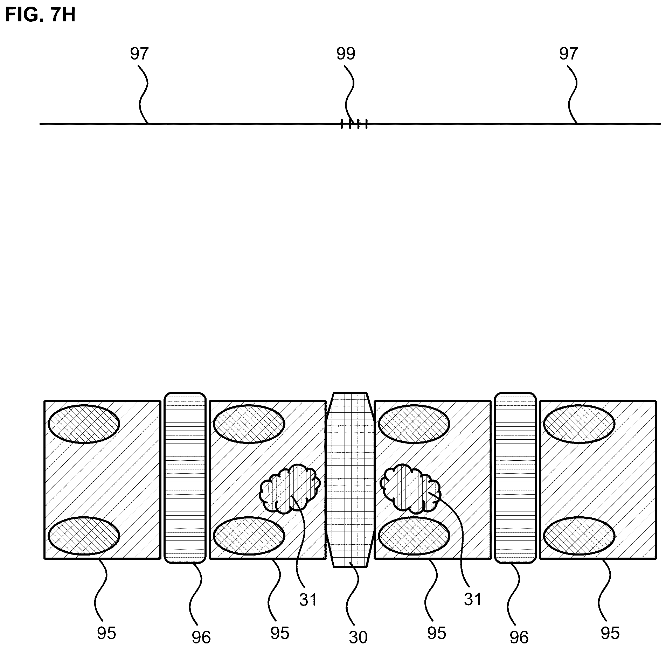

FIG. 7H is an explanatory drawing illustrating the eighth step (suturing of wound) of a surgical example using the treatment device according to the present invention.

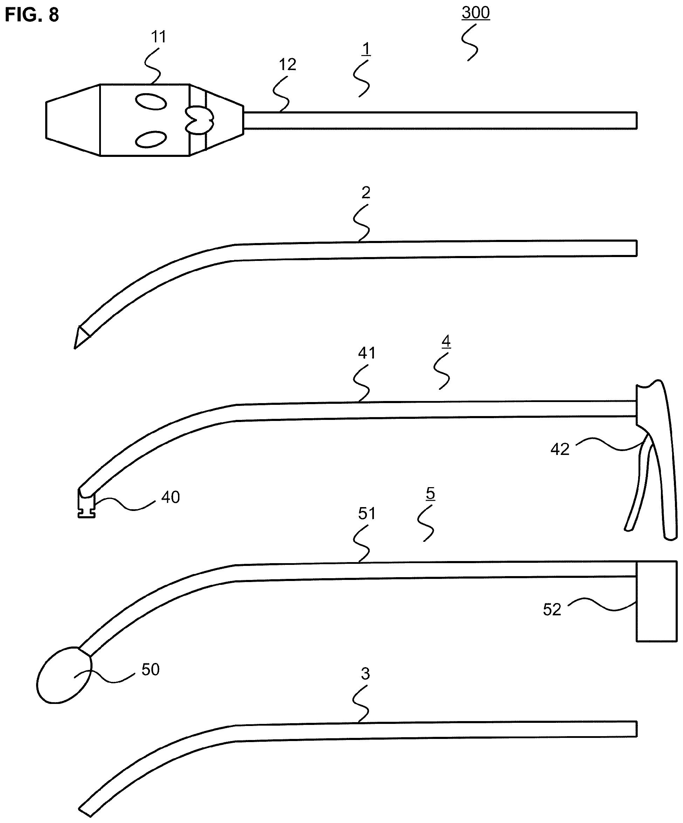

FIG. 8 is an explanatory drawing illustrating an example configuration of a treatment device, a third embodiment according to the present invention.

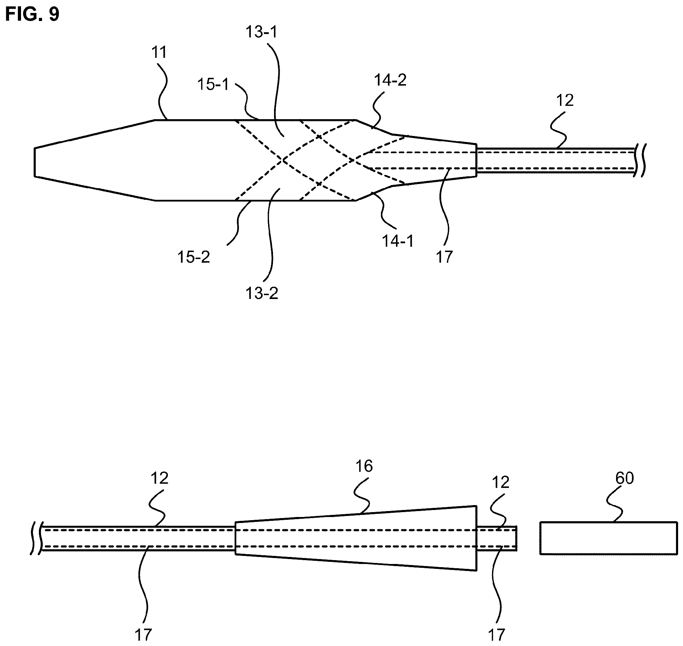

FIG. 9 is an explanatory drawing illustrating an example configuration of a trial cage for a treatment device, a fourth embodiment of the present invention.

### DETAILED DESCRIPTION OF THE INVENTION

### 1. Outline of Embodiment

First, an outline of a representative embodiment disclosed in the present application will be given. Reference signs in the drawings that are referred to in parentheses in the outline description of representative embodiment are merely illustrative of what is included in the concept of the components to which they refer.

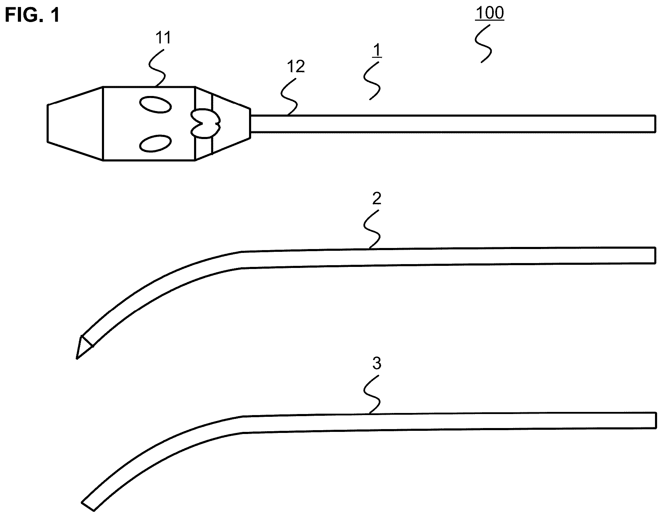

### [1] Trial cage to assist in injection of bone cement into vertebral body (FIG. 1 - FIG. 4)

A typical embodiment disclosed in this application is treatment device (100) including trial cage (1), bone drill needle (2), and bone cement filling tube (3), and is configured as follows. Here, bone cement filling tube (3) is a tube that is pre-filled with bone cement, and by applying pressure from the distal end, the filled bone cement can be discharged through an opening at the proximal end and filled into the affected area.

The trial cage includes head (11) of a predetermined thickness, width, and length, and support rod (12) that supports the head at a longitudinal end of the head.

The head includes a flat surface that defines the predetermined thickness and at least one guide hole (13-1).

The guide hole penetrates the head in the thickness direction from the distal side of the head toward one of flat surfaces and allows passage of respective tip sections of the bone drill needle and the bone cement filling tube.

This provides a treatment device used in procedures prior to the insertion of an artificial intervertebral disc into an intervertebral disc space that is capable of forming bone tunnels in vertebrae above and below the artificial intervertebral disc, filling bone cement through those tunnels while the same device is inserted, and reinforcing the vertebrae above and below a cage.

### [2] Further add vertebral body drill and balloon catheter (FIG. 8).

The treatment device of [1] further includes at least one of vertebral body drill (4) and a balloon catheter (5).
the vertebral body drill includes tube (41) and drilling end (40) at the proximal end of the tube configured so as to extend in the direction of extension of the tube, to be able to bend from the direction of extension, and to be able to pass through the guide hole in the direction of extension of the tube.
the balloon catheter includes tube (51), balloon (50) at a proximal end of the tube, and balloon pressurizer (52) at a distal end that can inflate the balloon, in which the balloon is able to pass through the guide hole in a deflated state.

This allows the vertebral body to be reinforced back to a more appropriate size when the vertebral body adjacent to the intervertebral disc space to be implanted has been damaged due to a compression fracture, etc., at the time of surgery to implant an artificial intervertebral disc, and the surgery to applied, for example, in conjunction with or in place of surgery to reconstruct that vertebral body.

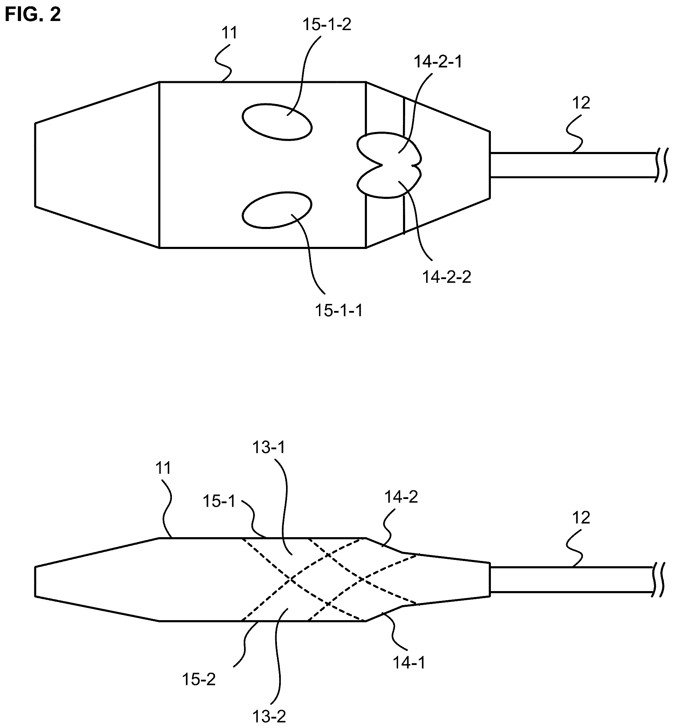

### [3] A plurality of guide holes (FIG. 1 and 2)

In the treatment device of [1] or [2], the head has a plurality of guide holes, including other guide holes (13-2) that penetrate from the other side to the one side.

This allows the respective tip sections of bone drill needle (2), bone cement filling tube (3), vertebral body drill (4) and/or balloon catheter (5) to protrude on both sides of trial cage (1).

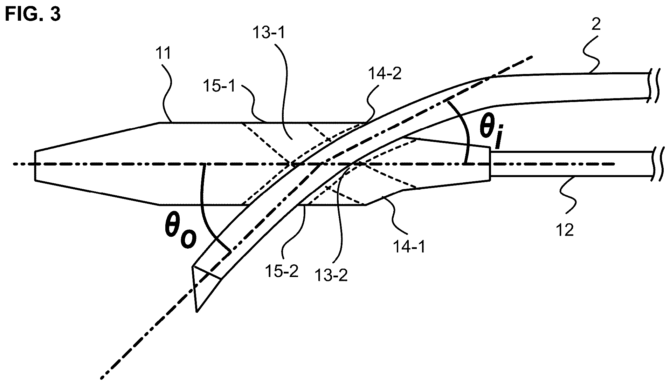

### [4] Bend in the direction of the vertebral body (FIG. 3)

In the treatment device of [1],[2] or [3], the guide hole (13-2) of the trial cage, with the side of the guide hole closer to the support rod as inlet (14-2) and the far side as outlet (15-2), is curved so that an protrusion angle (θₒ) is larger than the entry angle (θᵢ) of the inlet when an instrument other than the trial cage is inserted. The instrument (other than the trial cage) is curved to match the same (the guide hole). The entry angle (θᵢ) and the protrusion angle (θₒ) are specified, for example, as follows. That is, the entry angle (θᵢ) of the device is the angle at the inlet of the central axis of the device with respect to the central axis of support rod (12), and the protrusion angle (θₒ) is the angle at the outlet of the central axis of the device with respect to the central axis of support rod (12).

This allows the respective directions of bone drill needle (2), bone cement filling tube (3), vertebral body drill (4) and/or balloon catheter (5) protruding from trial cage (1) to be oriented toward the center of the vertebral body, which allows for a more appropriate bone cement filling site.

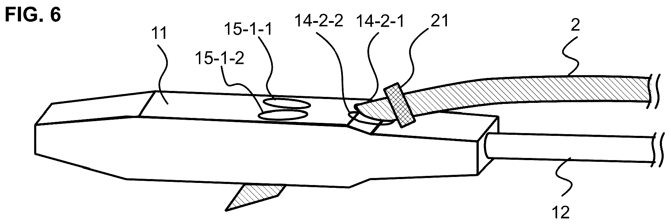

### [5] Stopper to prevent excessive protrusion (FIG. 5, FIG. 6)

In the treatment device of any one of [1] to [4], bone drill needle (2) includes stopper (21) that abuts against one side of the bone drill needle when bone drill needle (2) protrudes a predetermined length from the other side stops it from protruding any further.

This prevents accidents in which bone drill needle (2) protrudes excessively, even when the vertebral bone is fragile.

### [6] Guide hole and support rod communicate (FIG. 9)

In the trial cage of the treatment device of any one of [1] to [5], the support rod is hollow and communicates with the guide holes.

This allows bleeding near the head of the trial cage to be expelled from the operative field through the guide hole and through the hollow (through hole 17) in the support rod, thus ensuring a clear field of view.

### 2. Details of the embodiments

Details of the embodiments will be described in more detail.

### [Embodiment 1]

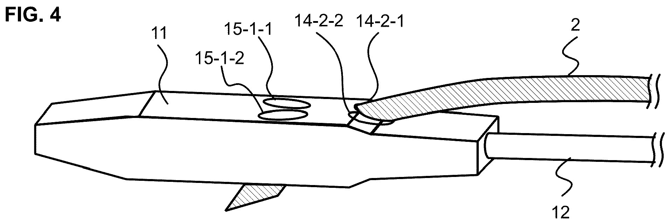

FIG. 1 is an explanatory drawing illustrating an example configuration of a treatment device 100 according to the present invention. FIG. 2 is an enlarged view of a tip section of trial cage 1, FIG. 3 is an explanatory drawing illustrating its configuration, and FIG. 4 is an explanatory drawing illustrating in perspective an action of the tip section. The top view is shown in the upper part of FIG. 2 and the side view is shown in the lower part. In the side view, guide holes 13-1 and 13-2 are shown in dashed lines, but are not visible from the actual side.
treatment device 100 includes trial cage 1, bone drill needle 2, and bone cement filling tube 3.

Bone drill needle 2 is an instrument with a sharp tip (proximal end), like a piercer, which can be used to drill a bone tunnel in the vertebral body bone by striking the distal end with a hammer or the like. If rotation is possible, the tip (proximal end) can be shaped as a drill rather than a piercer and the hole can be formed by rotation.

Bone cement filling tube 3 is a tube that can be pre-filled with bone cement, and by applying pressure from the distal end, the filled bone cement can be discharged through an opening at the proximal end and injected and filled into the affected area. The distal end has a syringe-like structure to allow pressure to be applied from the distal end, and the tip (i.e., proximal end) can be inserted into the created bone tunnel to deliver bone cement into the vertebrae.

Trial cage 1 includes head 11 of a predetermined thickness, width, and length, and support rod 12 that supports the head 11 at the end thereof. Head 11 includes a flat surface that defines the predetermined thickness and at least one guide hole 13-2. Guide hole 13-2 penetrates from one flat surface of head 11 (top surface in FIGS. 3 and 4) to the other (bottom surface in FIGS. 3 and 4) and is configured with a bore diameter that allows passage of the respective tip sections of bone drill needle 2 and bone cement filling tube 3. As illustrated in FIGS. 1 to 4, it is suitable if inlet 14-2 of guide hole 13-2 is formed not only on the flat surface of head 11 but also on a slightly distally inclined surface, penetrating from the distal side to one of the flat surfaces of the head, in the direction of the thickness of the head. In this document, even if the penetration is not only from a flat surface but also from a slightly inclined surface to the other surface, it may be expressed simply as "through from the top surface to the bottom surface" or "through from the bottom surface to the top surface". FIG. 3 shows an example of bone drill needle 2, but the same is true for bone cement filling tube 3, as well as for vertebral body drill 4 and balloon catheter 5 in Embodiment 3, described below.

This provides a treatment device used in procedures prior to the insertion of an artificial intervertebral disc into an intervertebral disc space that is capable of forming bone tunnels in vertebrae above and below the artificial intervertebral disc, filling bone cement through those tunnels while the same device is inserted, and reinforcing the vertebrae above and below the artificial intervertebral disc from inside thereof.

The thickness, width, and length of head 11 of trial cage 1 correspond to the thickness, width, and length of the artificial intervertebral disc that is inserted into the intervertebral disc space. Head 11 is provided in various sizes in suitable increments, for example, 6-14 mm thick, 14-24 mm wide, and 45-65 mm long, and is inserted and used to measure the patient's intervertebral disc space to determine the appropriate size of the artificial intervertebral disc to be implanted. The material is, for example, stainless steel or aluminum alloy.

It is suitable if head 11 of trial cage 1 has a plurality of guide holes. For example, as shown in FIGS. 2 and 3, in addition to guide hole 13-2 that penetrates from the top surface to the bottom surface described above, there are two guide holes 13-1 that penetrate from the bottom surface to the top surface, each in the left and right directions (up and down in the top view of FIG. 2). Here, the reference sign 13 is used to refer to the guide holes collectively, and branch numbers such as 13-1, 13-2, etc. are used to distinguish them. Also, for inlets and outlets, 14 and 15 are also used for collective reference, and branch numbers are added for distinction in their placement. There are two guide holes 13-2 that penetrate from the top surface to the bottom surface, one on each side of the axis of support rod 11, entering at inlets 14-2-1 and 14-2-2 on the top surface of head 11 -2 and penetrate to outlets 15-2-1 and 15-2-2 on the bottom surface of head 11. Although the bottom view is omitted in FIG. 2, 15-2-1 is located on the opposite side of outlet 15-1-1 in the top view, and 15-2-2 is located on the opposite side of outlet 15-1-2 in the top view. Similarly, there are two guide holes 13-1 that penetrate from the bottom surface to the top surface, one on each side of the axis of support rod 11, entering at inlets 14-1-1 and 14-1-2 on the bottom surface of head 11 -2 and penetrate to outlets 15-1-1 and 15-1-2 on the bottom surface of head 11. Although the bottom view is omitted in FIG. 2, the inlets 14-1-1 and 14-1-2 are located on the opposite sides of 14-2-1 and 14-2-2 in the top view, respectively. In addition, inlets 14-2-1 and 14-2-2, and inlets 14-1-1 and 14-1-2 may partially overlap, depending on the diameter and placement of the guide holes, as illustrated in the upper side of FIG. 2. The multiple guide holes may also intersect inside head 11 of trial cage 1. Desirably, inlet 14 of guide hole 13 should be chamfered. Bone drill needle 2 and bone cement filling tube 3 are guided more smoothly as they are inserted. Desirably, bone cement filling tube 3 should be thinner than bone drill needle 2. When inserted into the bone tunnel formed by bone drill needle 2, it is inserted smoothly without hitting the corners of the bone tunnel.

Bone drill needle 2 and bone cement filling tube 3 and guide holes 13-1, 13-2 are desirably each curved enough and in the same manner to allow the tip sections of bone drill needle 2 and bone cement filling tube 3 to penetrate guide holes 13-1 and 13-2. The direction of the curvature is shown in FIG. 3, with the side of guide hole 13-2 closer to support rod 12 as inlet 14-2 and the far side as outlet 15-2, and guide hole 13-2 and bone drill needle 2 are curved so that the protrusion angle (θₒ) at the outlet of bone drill needle 2 is larger than the entry angle (θᵢ) at the inlet of bone drill needle 2. The entry angle (θᵢ) and the protrusion angle (θₒ) are defined, for example, by the angle of the central axis of bone drill needle 2 at the inlet and outlet, respectively, relative to the central axis of support rod 12. Although bone drill needle 2 is used as an example, the same applies to bone cement filling tube 3 and other instruments described below.

This allows the respective directions of bone drill needle 2 and bone cement filling tube 3 protruding from trial cage 1 to be oriented toward the center of the adjacent vertebral body, which allows for a more appropriate bone cement filling site.

However, bone drill needle 2, bone cement filling tube 3, and guide holes 13-1 and 13-2 may be linear instead of curved. Even if the guide holes 13-1 and 13-2 are curved and the bone drill needle 2 and bone cement filling tube 3 are linear, their tips guide them to the inside of the adjacent vertebral body by making their diameters smaller than the those of the guide holes 13-1 and 13-2.

FIG. 2 and other examples show that head 11 of trial cage 1 is provided with two guide holes penetrating from the top surface to the bottom surface and two guide holes penetrating from the bottom surface to the top surface; however, a configuration with one guide hole for each is also suitable. This is because the two guide holes can be arranged so that they do not intersect inside head 11 of trial cage 1. By configuring the guide holes so that they do not overlap, it is possible to eliminate the risk of equipment, etc. getting caught in the intersecting areas.

Furthermore, head 11 of trial cage 1 may be configured with only one guide hole penetrating from one side to the other. By using a single guide hole, bone cement is filled into either the upper or lower vertebral body for each insertion of trial cage 1; however, trial cage 1 can be inserted one time each for the upper and lower vertebral body processes. Although the number of insertions increases, a single guide hole can be positioned along the central axis of head 11 of trial cage 1, which simplifies the procedure of bone drilling in the approximate center of the vertebral body.

### Examples of surgical procedures using the treatment device of the present invention

FIG. 7A through 7H are explanatory drawings illustrating example surgical procedures using the treatment device according to the present invention. FIGS. 7A through 7H show steps 1 through 8. The figures are depicted with the patient lying down and an incision made from the lateral abdomen to the affected area for implantation of an artificial intervertebral disc. Left and right in the figure are up and down for the patient, but the direction is expressed according to the illustration.

Step 1 (FIG. 7A): Incise the patient's lateral abdomen to reach the affected area, insert wound opener 90 into incision 98 and open to provide a field of view.

Step 2 (FIG. 7B): Insert Cobb 91 into the affected intervertebral disc 96-x and separate the damaged disc 96-x from vertebral body 95-3. Although not shown in the figure, Cobb 91 is also inserted in vertebral body 95-2 on the opposite side to separate and remove intervertebral disc 96-x.

Step 3 (FIG. 7C): Insert head 11 of trial cage 1 into the intervertebral disc space created by the removal of the affected intervertebral disc 96-x to determine the size of artificial intervertebral disc 30 to be finally implanted. Head 11 of trial cage 1 is pushed slightly up and down through the intervertebral disc space by inserting it strongly.

Step 4 (FIG. 7D): Insert bone drill needle 2 through the guide hole in head 11 of trial cage 1 and tap its tail with a hammer to drill vertebral body 95-3 to form a bone tunnel. Although not shown in the figure, the bone drill needle 2 is inserted for the other side as well, and vertebral body 95-2 is drilled to form a bone tunnel.

Step 5 (FIG. 7E): Insert bone cement filling tube 3 through the guide hole in head 11 of trial cage 1 into the bone tunnel in vertebral body 95-3 formed in the third step above, and inject and fill with bone cement 31. Although not shown in the figure, bone cement filling tube 3 is also inserted through the guide hole in head 11 of trial cage 1 into the bone tunnel in vertebral body 95-2 on the opposite side, and bone cement 31 is injected and filled.

Step 5 (FIG. 7F): Pull out trial cage 1.

Step 6 (FIG. 7G): Insert artificial intervertebral disc 30 into the intervertebral disc space.

Step 7 (FIG. 7H): remove the wound opener 90 and suture the incision.

This completes the surgical implantation of artificial intervertebral disc 30 into the spine.

Thus, with trial cage 1 inserted, the vertebrae in the left and right (up and down in the patient) directions can be drilled and bone cement injected into the vertebral body. The trial of PTL 1 allows various bone growth promoting substances, such as bone cement, to be introduced into the affected area through the tip section, which promotes fusion with the artificial intervertebral disc to be implanted, but does not strengthen the vertebral body from its inside; for patients with osteoporosis, there remains concern about the settling of the artificial intervertebral disc into the vertebrae and future compression fractures of the upper and lower vertebral bodies. In contrast, according to the surgical example shown in FIG. 7, bone cement is filled inside the vertebrae on both sides of the artificial intervertebral disc 30 to be implanted, which reinforces the vertebrae themselves and greatly reduces the risk of future settlement of the artificial intervertebral discs into the vertebrae or compression fractures. In addition, with trial cage 1 inserted, the vertebrae can be drilled in the left and right (up and down in the patient) directions and bone cement can be injected directly, which not only shortens the operation time, but also allows bone cement filling tube 3 in the fifth step to be accurately guided into the bone tunnel formed in the fourth step since the same guide hole is used to inject the bone cement.

### [Embodiment 2]

FIG. 5 is an explanatory drawing illustrating an example configuration of a treatment device 200, a second embodiment according to the present invention, and FIG. 6 is an explanatory drawing illustrating in perspective an action of tip section of trial cage 1 thereof. Trial cage 1 and bone cement filling tube 3 may be the same as trial cage 1 and bone cement filling tube 3 of treatment device 100 of Embodiment 1 shown in FIGS. 1 to 4. Bone drill needle 2 is equipped with stopper 21. When bone drill needle 2 is inserted into one of guide holes 13, stopper 21 stops the insertion when bone drill needle 2 protrudes from the outlet 15 of guide hole 13 to a predetermined length, by abutting against head 11 of trial cage 1 for example at inlet 14 to prevent bone drill needle 2 from protruding further.

This prevents accidents in which bone drill needle 2 protrudes excessively, even when, for example, the vertebral bone is fragile.

In this Embodiment 2, the example of attaching stopper 21 to bone drill needle 2 is shown, but the stopper may be attached to the cement filling tube 31 or other instruments (i.e., vertebral body drill 4 and/or balloon catheter 5) described below in the same manner.

### [Embodiment 3]

FIG. 8 is an explanatory drawing illustrating an example configuration of a treatment device 300, a third embodiment according to the present invention. Vertebral body drill 4 and balloon catheter 5 are added to the first embodiment of treatment device 100. Vertebral body drill 4 and balloon catheter 5, like bone drill needle 2 and others, are shaped to allow the tip section to pass through guide hole 13 in head 11 of trial cage 1. Vertebral body drill 4 has tube 41, drill end 40 at the tip (proximal end) of tube 41, and handle 42 at the hand (distal end), and is configured so that by operating the handle 42, the drill end 40 can be bent from the same direction as the tip section of the tube 41 to a right angle direction. Balloon catheter 5 has tube 51, balloon 50 at its tip (proximal end) and balloon pressurizer 52 at its hand (distal end), and is configured so that balloon 50 can be inflated by injecting, for example, contrast medium from balloon pressurizer 52 through tube 51. Balloon pressurizer 52, includes, for example, a syringe for injecting contrast medium into balloon 50 and a pressure gauge that measures the internal pressure of balloon 50.These are simplified into a block in FIG. 8.

With treatment device 300, several steps can be added to the surgical examples described by citing FIGS. 7A through 7H to achieve better treatment in cases where there is a compression fracture of the vertebral body due to osteoporosis or other factors. For example, after bone drilling in FIG. 7D, vertebral body drill 4 is inserted through guide hole 13 in trial cage 1 and drill end 40 is guided through the bone tunnel formed in FIG. 7D into the vertebral body. balloon catheter 5 is then inserted through guide hole 13 in trial cage 1, balloon 50 is guided into the vertebral body through the formed bone tunnel, and balloon 50 is inflated within the vertebral body using balloon pressurizer 52 to create a cavity in the vertebral body. Returning to the fifth step shown in FIG. 7E, bone cement filling tube 3 is inserted through the guide hole in head 11 of trial cage 1, and the cavity formed by inflation of balloon 50 is filled with bone cement 31.

This allows the vertebral body to be reinforced back to a more appropriate size when the vertebral body adjacent to the intervertebral disc space to be implanted has been damaged due to a compression fracture, etc., at the time of surgery to implant an artificial intervertebral disc, and the surgery to applied, for example, in conjunction with or in place of surgery to reconstruct that vertebral body.

Although the example of adding both vertebral body drill 4 and balloon catheter 5 to treatment device 300 is shown in Embodiment 3, only one or even other device may be added as necessary. Bone drill needle 2 of treatment device 300 may be provided with stopper 21 shown in Embodiment 2, and vertebral body drill 4, balloon catheter 5, and bone cement filling tube 3 may also be provided with their own stopper, as appropriate.

### [Embodiment 4]

FIG. 9 is an explanatory drawing illustrating an example configuration of trial cage 1. In trial cage 1, support rod 12 is hollow, and is configured such that through hole 17 including the hollow portion of support rod 12 communicates with guide hole 13 of head 11. When a plurality of guide holes 13 intersect, through hole 17 should communicates with the intersection as illustrated in FIG. 9. That is, through hole 17 of support rod 12 leads from guide hole 13, such as at the intersection, to the end of support rod 12.

This allows bleeding near head 11 of trial cage 1 to be expelled from the operative field through guide hole 13 and through the hollow (through hole 17) in the support rod, thus ensuring a clear field of view.

For example, as shown in FIGS. 4, 6, and 7D, when the bone drill needle 2 is thrust into the vertebral body through guide hole 13 in head 11 of trial cage 1 to form a bone tunnel, bleeding from the bone tunnel may flood the intervertebral disc space and cover head 11 of trial cage 1, making inlet 14 of guide hole 13 invisible. Then, when the next attempt is made to insert bone cement filling tube 3 into guide hole 13, it is difficult to see inlet 14, which makes insertion difficult. In contrast, the field of vision can be restored by attaching suction tube 60 to the far end of support rod 12 of trial cage 1 and suctioning and draining blood from inlet 14 and outlet 15 of guide hole 13, as illustrated in FIG. 9.

Furthermore, as illustrated in FIG. 9, it is more suitable to provide grip 16 on support rod 12. Grip 16 should be provided near the relatively distal end of support rod 12, leaving enough room for suction tube 60 to be attached. With trial cage 1 supported by holding this grip 16, suction tube 60 can be attached.

The present embodiment can be combined with any of the other embodiments.

While the present invention made by the present inventor has been described in detail with respect to the embodiments thereof, the present invention is not limited to these embodiments. Various changes may be made without departing from the spirit and scope of the present invention.

### Industrial Applicability

The present invention the present invention relates to a treatment device for a spine which inserts an intervertebral spinal implant (i.e., an artificial intervertebral disc) into an intervertebral disc space, and is particularly suitable for use in surgery to insert it between vertebrae before the artificial intervertebral disc is inserted to secure a gap (i.e., intervertebral disc space) for insertion.

### EXPLANATION OF SIGN

1 Trial cage
2 Bone drill needle
3 Bone cement filling tube
4 Vertebral body drill
5 Balloon catheter
11 Head of trial cage
12 Support Rod of trial cage
13 Guide hole
14 Inlet of guide hole
15 Outlet of guide hole
16 Grip
17 Through hole
21 Stopper
30 Artificial intervertebral disc
31 Bone cement
40 Drill end
41 Tube
42 Handle
50 Balloon
51 Tube
52 Medical balloon pressurizer
60 Suction Tube
90 Wound opener
91 Cobb
95 Vertebral body
96 Intervertebral disc
97 Body surface
98 Incision
99 Suture section
100, 200, 300 treatment device

## Claims

1. A treatment device comprising a trial cage, a bone drill needle, and a bone cement filling tube, wherein
the trial cage has a head of a predetermined thickness, width, and length, and a support rod that supports the head at the longitudinal end of the head,
the head has a flat surface that defines the predetermined thickness and at least one guide hole, and
the guide hole penetrates the head in the thickness direction from the distal side of the head toward one of a flat surfaces and allows passage of respective tip sections of the bone drill needle and the bone cement filling tube.

2. The treatment device of claim 1, further comprising at least one of a vertebral body drill and a balloon catheter, wherein
the vertebral body drill includes a tube of the vertebral body drill and a drilling end at the proximal end of the tube configured so as to extend in the direction of extension of the tube, to be able to bend from the direction of extension, and to be able to pass through the guide hole in the direction of extension of the tube of the vertebral body drill, and
the balloon catheter includes a tube of the balloon catheter, a balloon at a proximal end of the tube, and a balloon pressurizer at a distal end that can inflate the balloon, and wherein the balloon is configured to be able to pass through the guide hole in a deflated state.

3. The treatment device according to claim 1 or 2, wherein
the head has a plurality of guide holes, including other guide holes that penetrate from the other side to the one side.

4. The treatment device according to claim 1 or 2, wherein
the guide hole of the trial cage, with the side of the guide hole closer to the support rod as an inlet and the far side as an outlet, is curved so that an outlet protrusion angle is larger than the entry angle when an instrument other than the trial cage is inserted into the guide hole, and the instrument other than the trial cage is curved to match the curvature.

5. The treatment device according to claim 1 or 2, wherein
the bone drill needle includes a stopper that abuts against one side of the bone drill needle when the bone drill needle protrudes a predetermined length from the other side stops it from protruding any further.

6. The treatment device wherein in the trial cage according to claim 1 or 2,
the support rod is hollow and communicates with the guide holes.
